# EUROPEAN PATENT SPECIFICATION

(11) **EP 3 288 755 B1**
(45) Date of publication and mention of the grant of the patent: **14.06.2023**
(21) Application number: 15890186.8
(22) Date of filing: 29.04.2015
(51) Int. Cl.: B32B 5/12, A47C 21/00, A47G 9/02, A61F 13/00, A61G 7/057, B32B 7/02, D06M 17/00

(54) **MULTI-LAYERED FABRIC**
MEHRSCHICHTIGER STOFF
TISSU MULTICOUCHE

(43) Date of publication of application: 07.03.2018
(73) Proprietor: Oratex Inc., Anjou, Québec H1J 1L8 (CA)
(72) Inventor: MAROTTA, Andy, Montréal, Québec H1E 5E5 (CA)
(74) Representative: Nederlandsch Octrooibureau
(86) International application number: PCT/CA2015/050360
(87) International publication number: WO 2016/172782

(56) References cited:
- EP-A1- 2 319 474
- EP-A1- 2 319 474
- WO-A1-96/00548
- WO-A1-98/42290
- WO-A1-2009/111822
- WO-A1-2013/099635
- WO-A1-2014/036472
- CA-A1- 2 483 773
- JP-U- H0 562 671
- US-A- 4 944 060
- US-A- 5 926 884
- US-A1- 2008 098 532
- US-A1- 2010 069 863
- US-A1- 2013 025 053

## Description

### FIELD OF THE INVENTION

The present invention relates to a multi-layered fabric and bed pad.

### BACKGROUND OF THE INVENTION

Hospitals and nursing homes treating long-care immobilized patients are faced with the ancillary medical issue of decubitus ulcers which may be developed by such patients. Decubitus ulcers are more commonly known as bed sores.

Bed sores develop in patients who are unable to move and thus constantly have a part of their body, such as their back or the back of their legs, in contact with a support surface, such as a bed, brace, or chair. A continuous contact of a part of the body with such a surface can cause humidity, heat, moisture, and fluid build-up between the support and the skin of the patient which is known to create a breeding ground for bacteria. Such a build-up of humidity, moisture, and fluid may further cause tissue in the area of the build-up to weaken and tear. This weakening, in combination with the pressures applied to the subcutaneous tissues of the skin can lead to the development of ulcers, or bed sores. In certain untreated cases, the ulcer may become infected and discharge exudates tending to further increase the moisture level around the ulcer and aggravate the infection and ulcer. Without pressure relief or aeration of the body part, the ulcer is unable to heal, or may continue to worsen into a chronic condition.

Typically, patients with bed sores are confined to a bed. When pressure points between the bed and the body party arise, reduced blood flow to the pressured body area and/or disintegration and necrosis of epithelial tissue may follow. When the patient is shifted in position to relieve pressure or provide aeration to the body part, tears in the weakened skin may occur. Healing is thus not possible without medical intervention which may include a constant repositioning of the body part, for example an alternation between a person's side, requiring hospital staffing attention and resources.

For such type of wound management, dressings are the primary option available to medical staff for managing ulcers and ulcer exudates. An example of overlay for a patient support such as a sleeping and/or seating surface (e.g. a hospital bed, mattress etc.) is shown in EP2319474. While dry dressings are known in the art to prevent bacterial propagation into the open wound, once the dressing becomes sodden, bacteria can in fact use the dressing to propagate to the ulcer. Furthermore, a sodden dressing further causes the skin surrounding the ulcer to become wet from exudates, which can cause excoriation of the area when the patient shifts position, leading to pain and discomfort to the patient.

There exists in the prior art various materials used to create pads designed to both absorb fluid while simultaneously providing pressure relief to an ulcered area to facilitate healing. Various combinations of materials, such as foam, sheet foams, cavity foams, knitted dressings formed from cotton, polyester or viscose fibres or fabrics, or other fibrous materials, sheet hydrogels, are layered to create a structure, such as a pad, having ulcer treatment properties, such as fluid absorption, support and aeration properties. For example, to provide aeration to the wound, such layered configurations are designed to create channels for air flow below the skin while simultaneously providing support to the body part.

One drawback of such prior art pads is that although the layers of foams, cotton, viscose or polyester textiles are able to retain fluid within internal spaces formed within their structure similar to the structure of a sponge, when such materials are placed under pressure, for example under the weight of a patient, fluid may be released from the spaces and leak from the pad. Pads which provide fluid retention properties, such as hydrophilic materials, may avoid such leaking.

Another drawback of such absorbent padding is that their multi-layered material structures also retain exudates which may block the internal spaces and prevent air flow from aerating the wound. Furthermore, it may be difficult to thoroughly clean such materials through washing due to densely and randomly packed internal spaces which form spaces or cavities which are very fine, randomly and densely packed, tending to firmly embed dried exudates therein.

Furthermore, the use of materials which are fluid absorptive, such as foam or hydrophilic treated fibres absorb and retain moisture near the skin of a patient which can maintain a high humidity level adjacent to the skin tending to promote degradation and weakening of the area. Still furthermore, the use of materials with such fluid absorptive properties make washing of the material more difficult, since the fluid absorption can render the material heavier and difficult to handle for cleaning staff, as well as lead to increased drying times.

The advent of three-dimensional (3D) spacer fabric, which is commonly known to provide a structure with dual breathability and compression/recovery resistance characteristics, provides an interesting material for ulcer treatment. Indeed, three-dimensional spacer materials or fabrics are known to be beneficial for wound management therapy in which the wound dressing itself acts as a waste canister to collect and store wound exudates removed from a wound site, while providing improved air circulation compared to traditional materials such as an absorbent pad of a fibrous material discussed hereinabove.

However, one drawback of prior art applications of spacer fabric for the treatment of ulcers is that existing bed pads employing spacer fabrics tend to absorb or pool fluid within the spacer material itself which tends to block the air circulation properties of the air channels formed by the spacer material. Since the spacer material closest to the skin becomes sodden, the spacer fabric prevents fluid from moving towards the base of the pad and away from the ulcer, thereby blocking air circulation channels provided adjacent to the skin. As a result, existing bed pads employing spacer fabric do not provide airflow and breathability immediately below the skin, a drawback similar to the fibrous materials.

Therefore, what is needed is an inexpensive reusable multi-layered fabric for use in padding for treating ulcers and the like which is effective in absorbing fluids, distributing the pressure of a patient's body and providing aeration of the area of the wound. As well, what is needed is multi-layered fabric that is also easy to wash and dry, and which can be adapted for different wound management therapies.

### SUMMARY OF THE INVENTION

In order to overcome these and other drawbacks, there is provided a multi-layered fabric for use in padding for treating ulcers, the fabric including at least one first layer including a first sublayer made of permeable material, a second sublayer made of permeable material, and a third sublayer located between the first and the second sublayers, the third sublayer including a plurality of fibers interconnecting the first sublayer with the second sublayer for allowing an airflow between the first and second sublayers, and at least one second layer made of impermeable material disposed in parallel and secured to the at least one first layer, wherein the density of the plurality of fibers fibres allows a vertical airflow of 140 cubic centimeters per square centimeter of surface area, the airflow being a vertical airflow through the first sublayer and the second sublayer ;and wherein the first and second sublayers comprise bristles protruding from a first sublayer surface and a second sublayer surface for providing frictional engagement with the bristles of an adjacent sublayer.

In one possible embodiment, the plurality of fibers are configured to form air channels allowing air flow to flow between the first sublayer and the second sublayer.

In another possible embodiment, the first sublayer and the second sublayer comprise holes allowing air flow and liquid to flow there through.

In another possible embodiment, the first sublayer and second sublayer are made of a flexible material.

In another possible embodiment, the multi-layered fabric further includes at least one third layer of permeable material disposed in parallel and secured to the at least one first layer opposite to the at least one second layer.

In another possible embodiment, the third layer is a polyester material.

In another possible embodiment, the third layer includes a patterned surface.

In another possible embodiment, the first and second sublayers are formed from a polyester material.

In another possible embodiment, the first and second sublayers are formed from a microfiber fabric.

In another possible embodiment, the first and second sublayers are formed from a microfilament fabric.

In another possible embodiment, the first and second sublayers are hydrophobic.

In another possible embodiment, the at least one second layer comprises two second layers.

In another possible embodiment, the at least one second layer is formed from nylon.

In another possible embodiment, the at least one first layer is a three dimensional spacer fabric.

In another possible embodiment, the plurality of fibers are disposed perpendicularly to the first sublayer and the second sublayer.

In another possible embodiment, the pluralities of fibers each comprise a bend.

In another possible embodiment, the plurality of fibers are aligned such that the fibers bend in a common direction.

In another possible embodiment, the plurality of fibers are disposed in pairs of fibers.

In another possible embodiment, the pairs of fibers are twisted.

In another possible embodiment, the plurality of fibers are made of nylon.

In another possible embodiment, the plurality of fibers are made of polyester.

In another possible embodiment, the fibers are a monofilament fiber.

As claimed , the density of the plurality of fibres allows a vertical airflow of 140 cubic centimeters per square centimeter of surface area.

In another possible embodiment, the first sublayer and the second sublayer are spaced apart by a distance of between three to twelve millimeters.

In another possible embodiment, the first sublayer and the second sublayer are spaced apart by a distance that varies over the area of the at least one first layer.

In another possible embodiment, the plurality of fibers are hydrophobic.

In another possible embodiment, the plurality of fibers are chemically treated to render the plurality of fibres hydrophobic.

In another possible embodiment, the plurality of fibers are knitted to the first sublayer and the second sublayer.

In another possible embodiment, the bristles are formed in part by a portion of the fibers.

In another possible embodiment, the at least one first layer comprises three first layers.

In another possible embodiment, each first layer has a different thickness than an adjacent first layer.

In another possible embodiment, each first layer has an increasing thickness to an adjacent first layer wherein the first layer adjacent the second layer has a lessor thickness than a first layer opposite the second layer.

In another possible embodiment, the fabric is machine washable.

In another possible embodiment, the at least one second layer extends to overlap with the at least one first layer.

In another possible embodiment, the at least one first layer is secured to the at least one second layer by stitching.

In another possible embodiment, the at least one first layer comprises a first layer periphery and the at least one second layer comprises a second layer periphery, wherein the first layer periphery and the second layer periphery are secured together by stitching.

In another possible embodiment, the first layer periphery and the second layer periphery are compressively stitched together.

In another possible embodiment, the multi-layered fabric further includes a breathable fabric peripheral cap secured to the first layer periphery and the second layer periphery.

In another possible embodiment, a bed pad is provided that includes the multi-layered fabric, wherein the bed pad includes one or more outer pad peripheries.

In another possible embodiment, the bed pad further includes at least one elastic band extending between the one or more outer pad peripheries.

In another possible embodiment, the bed pad includes a sheet of material secured to the one or more outer pad peripheries to engage a periphery of a bed.

In another possible embodiment, the bed pad further includes one or more fourth layers, each fourth layer including a fifth layer including the first layer, and a sheet of material including a sheet outer periphery and a sheet inner periphery, wherein the sheet outer periphery is connected to the at least one first layer and the sheet inner periphery is connected to the fifth layer, wherein the one or more fourth layers is disposed in parallel to the at least one first layer to form protrusions matching the curves of a human body.

In another possible embodiment the one or more fourth layers are provided to form protrusions running longitudinally the length of the bed pad.

In another possible embodiment, the bed pad is sized to be larger than the surface of a hospital bed such that a portion of the bed pad overhangs the bed.

In another possible embodiment, two or more bed pads are provided with each including the multi-layered fabric, wherein each of the two or more bed pads are secured together to create a foldable pad.

### BRIEF DESCRIPTION OF THE FIGURES

FIG. 1 is a top view of a bed pad employing a multi-layered fabric, in accordance with an illustrative embodiment of the invention;
FIG. 2 is a side cross-sectional view of the multi-layered fabric taken along the lines 2-2 of FIG. 1;
FIG. 3 is a side cross-sectional view of a first sub-layer shown in FIG. 2;
FIG. 4 is a top perspective view of the first sub-layer of FIG. 3;
FIG. 5 is a top cross-sectional view of a first sub-layer taken along the lines 3-3 of FIG. 3 showing the air flow channels provided in perpendicular configurations;
FIG. 6 is a top cross-sectional view of a first sub-layer taken along the lines 3-3 of FIG. 3 showing the air flow channels provided in a radial configuration;
FIG. 7 is a top cross-sectional view of a first sub-layer taken along the lines 3-3 of FIG. 3 showing density zone distributions of fibres;
FIG. 8 is a top cross-sectional view of a first sub-layer taken along the lines 3-3 of FIG. 3 showing density zone distributions of fibres;
FIG. 9 is a schematic view of a sub-layer of a multi-layered fabric, in accordance with an illustrative embodiment;
FIG. 10 is a schematic view of a sub-layer of a multi-layered fabric, in accordance with an illustrative embodiment;
FIG. 11 is a schematic view of a sub-layer of a multi-layered fabric, in accordance with an illustrative embodiment;
FIG. 12 is a side cross-sectional view of a multi-layered fabric taken along the lines 12-12 of Figure 1, ;
FIG. 13 is a side cross-sectional view of a multi-layered fabric taken along the lines 12-12 of Figure 1;
FIG. 14 is a side cross-sectional view of a multi-layered fabric taken along the lines 12-12 of Figure 1;
FIG. 15 is a side cross-sectional view of a multi-layered fabric taken along the lines 2-2 of Figure 1;
FIG. 16 is a side cross-sectional view of a multi-layered fabric taken along the lines 2-2 of Figure 1;
FIG. 17 is a top perspective view of a bed pad, in accordance with an illustrative embodiment of the invention;
FIG. 18 is a side cross-sectional view of a multi-layered fabric taken along the lines 2-2 of Figure 1;
FIG. 19 is a top view of a bed pad with an integrated pillow, in accordance with an illustrative embodiment of the invention;
FIG. 20 is a side cross-sectional view of a multi-layered fabric taken along the lines 2-2 of Figure 1, ;
FIG. 21 is a side cross-sectional view of a multi-layered fabric taken along the lines 2-2 of Figure 1;
FIG. 22 is a side cross-sectional view of the multi-layered fabric of FIG. 21;
FIG. 23 is a side cross-sectional view of a bed pad and bed taken along the lines 2-2 of Figure 1; and
FIG. 24 is a side cross-sectional view of a multi-layered fabric taken along the lines 2-2 of Figure 1.

### BRIEF DESCRIPTION OF THE PREFERRED EMBODIMENT

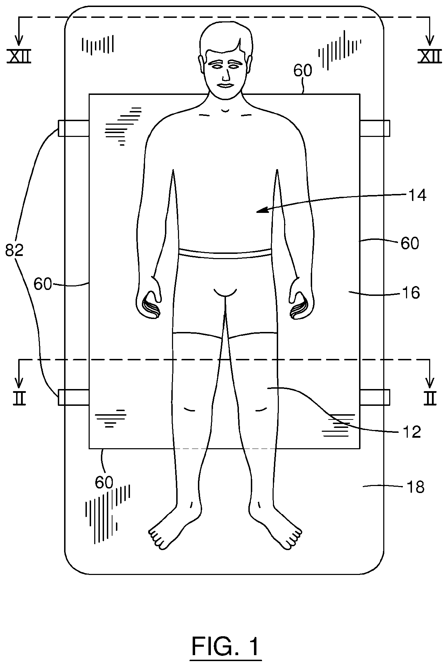

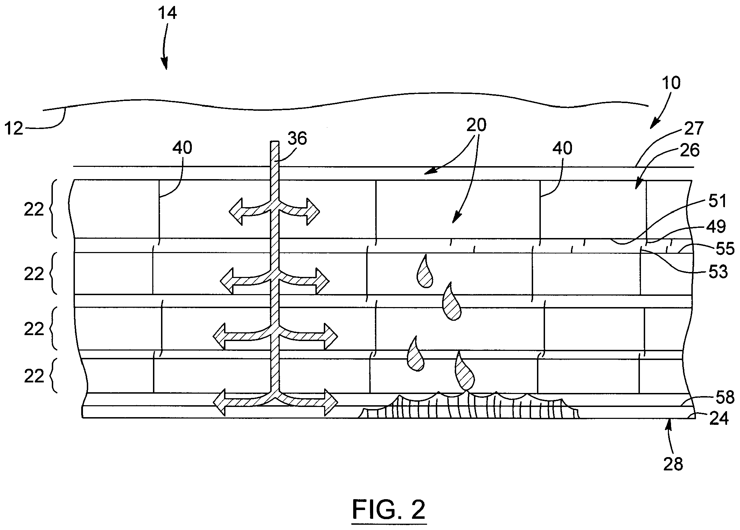

Now referring to FIGS. 1 and 2, there is provided a multi-layered fabric generally referred to by the reference numeral 10. The multi-layered fabric 10 allows air flow through and between the multi-layered fabric 10 for providing air circulation beneath the skin 12 of a body 14 of a patient when resting upon or in contact with the multi-layered fabric 10. In accordance with an illustrative embodiment, the multi-layered fabric 10 can be fabricated to form a bedding pad 16 for providing support to the body 14 of a patient when the bedding pad 16 is positioned on top of a bed 18 as will be described in more detail herein below.

Still referring to FIGS. 1 and 2, the structure of the multi-layered fabric 10 also allows moisture, fluid, humidity, and exudates, such as bodily fluids 20 secreted from an ulcer (not shown) or other wound on the skin 12, to be collected and pooled away from the skin 12 of the body 14. Once pooled away from the skin 12, the fluid 20 is allowed to evaporate and any residue, such as plasma, which solidifies may be collected and retained by the multi-layered fabric 10. The structure of the multi-layered fabric 10 promotes evaporation of moisture and humidity, away from the skin 12 to remove the basis for the growth of bacteria which can be harmful to the skin of the body 14, provides a structure to collect exudates, as well as provides air circulation for ensuring an aeration of the skin 12. The structure of the multi-layered fabric 10 also provides support and pressure relief to the skin 12 in a manner as will be described herein below.

Still referring to FIG. 1 and FIG. 2, the multi-layered fabric 10 includes at least one first layer 22 made of a permeable material and at least one second layer 24 made of impermeable material disposed in parallel and secured to the at least one first layer 22. In the illustrated embodiment, there is shown a multi-layered fabric 10 including four first layers 22 and two second layers 24, but other combinations of first layers 22 and second layers 24 may be provided. For example, a multi-layered fabric 10 including three first layers 22 and one second layer 24 may be provided. The choice of layers and configurations will depend on the desired moisture retention/evaporation properties, the support qualities, as well as the application, as will be discussed in more detail herein below. The upper most first layer 23, that is the first layer 22 which will be provided adjacent to the skin 12, will define a top layer 26 which will be in contact with the skin 12, while lower most second layer 24 will define a bottom layer 28 positionable adjacent to the bed 18 and which will be in contact with the bed 18. In one embodiment the top layer 26 may be provided with a coloured, patterned or textured fabric for aesthetic purposes. In another embodiment, a third layer 27, such as a sheet of material, may be provided adjacent to the top layer 26.

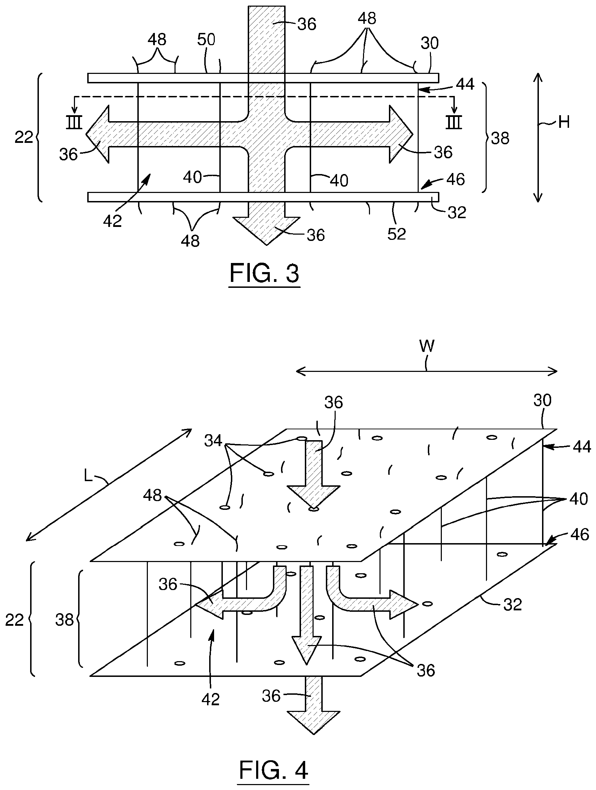

Now referring to FIG. 3 and FIG. 4, in addition to FIG. 2, the at least one first layer 22 includes a first sub-layer 30 made of permeable material and a second sub-layer 32 made of permeable material. For example, the first sub-layer 30 and second sub-layer 32 may be made of a lightweight, breathable, flexible, anti-microbial, non-cotton material that has moisture wicking properties. For example, the first sub-layer 30 and second sub-layer 32 may be stretchable in at least two directions to allow for some give in the multi-layered fabric 10 when subjected to a lateral loading. The first sub-layer 30 and second sub-layer 32 may be hydrophobic, and chemically treated with a fluid repellant. Optionally, the first sub-layer 30 and the second sub-layer 32 may be formed from materials based on a combination of cellulose and synthetic fibres which may be knitted to create air holes 34 to further enhance air permeability, moisture vapour permeability and wicking of fluid 20 and exudates through the first sub-layer 30 and second sub-layer 32. For example, the air holes 34 may be formed by knitting a material such that the first sub-layer 30 and the second sub-layer 32 form a mesh structure, as is commonly known in the art, such that the apertures in the mesh define the air holes 34. The first sub-layer 30 and a second sub-layer 32 therefore allow an airflow 36 to move through each sub-layer 30, 32 and also through the first layer 22 and onwards to an adjacent first layer 22.

Still referring to FIGS. 1, 2, 3 and 4, the at least one first layer 22 also includes a third sub-layer 38 located between the first sub-layer 30 and the second sub-layer 32. The third sub-layer 38 includes a plurality of fibres 40 interconnecting the first sub-layer 30 with the second sub-layer 32 for maintaining the first sub-layer 30 and the second sub-layer 32 in a spaced relationship to define a space 42 there between to allow the airflow 36 to move horizontally between the first sub-layer 30 and second sub-layer 32, and also to allow the airflow 36 to move vertically through the first sub-layer 30 and the second sub-layer 32. The fibres 40 may be hydrophobic, and chemically treated with a fluid repellant. Fluid 20 from the skin 12 may enter the multi-layered fabric 10 by diffusion through capillary action or 'wicking', and also under the force of gravity, and be drawn into the space 42 defined between the first sub-layer 30 with the second sub-layer 32 where it may be subjected to airflow 36 to promote evaporation thereof, or further be collected towards the bottom layer 28, atop of the at least one second layer 24.

The fibres 40 thus support the first sub-layer 30 and the second sub-layer 32 at a predetermined distance apart from one another. Optionally, the distance between the first sub-layer 30 and the second sub-layer 32 may be constant over the width (W) and length (L) of the multi-layered fabric 10, or the distance between the first sub-layer 30 and the second sub-layer 32 may be variable in a manner as will be described hereinbelow.

Each fibre 40 has a top fibre end 44 coupled to the first sub-layer 30 and a bottom fibre end 46 coupled to the second sub-layer 32. Optionally, each fibre 40 may be individually connected to the first sub-layer 30 at its top fibre end 44 and connected to the second sub-layer 32 at its bottom fibre end 46. Optionally, the fibres 40 may be integrated with the first sub-layer 30 and second sub-layer 32 by weaving, for example when the first sub-layer 30 and second sub-layer 32 are being formed. Optionally, each fibre 40 may form part of a continuous strand of material which is knitted with the first sub-layer 30 and the second sub-layer 32 during a knitting process so as to position and secure the fibres 40 with the first sub-layer 30 and a second sub-layer 32 at desired locations, for example in the case where the first layer 22 is a spacer material, as will be described hereinbelow. Illustratively, the fibres 40 are disposed vertically, or perpendicular to the parallel planes of the first sub-layer 30 and the second sub-layer 32.

As presently claimed, the first sub-layer 30 and the second sub-layer 32 includes bristles 48 which protrude from a first sub-layer top surface 50 and a second sub-layer bottom surface 52 for providing frictional engagement with the bristles 48 of an adjacent sub-layer. For example, bristles 49 provided on the first sub-layer bottom surface 51 may engage with bristles 53 provided on first sub-layer top surface 55 A bristle 48 may be formed from the top fibre end 44 and the bottom fibre end 46 projecting through the first sub-layer 30 and the second sub-layer 32, respectively, or may be provided and integrating separately to the first sub-layer 30 second sub-layer 32 itself, for example by being integrated during the knitting process of the first sub-layer 30 and the second sub-layer 32.

The fibres' 40 interconnection with the first sub-layer 30 and second sub-layer 32 form a spring action that translates into a support and cushioning effect when the first sub-layer 30 and the second sub-layer 32 are subjected to a compressive loading, for example when the first layer 22 is compressed under the weight of the body 14 when resting upon the bed 18 as shown in FIG 1. When subjected to a load, the fibres 40 will bend or deflect in response, and will provide a counteractive resistance which will maintain the first sub-layer 30 and second sub-layer 32 in a spaced relationship. Depending upon the loading, the first sub-layer 30 and second sub-layer 32 may be brought together due to a bending of the fibres 40. Upon relief from the loading, the fibres 40 will resiliently spring back to their initially biased shape. Optionally, each fibre 40 may have a biased shape which causes the fibres 40 to vertically or perpendicularly extend relative to the plane of the first sub-layer 30 and the second sub-layer 32, as illustrated. Optionally, each fibre 40 may have a biased shape, such as a curve to form an arch shape.

In accordance with an embodiment, the at least one first layer 22 may be formed from a spacer fabric material, such as a 3D knitted or 3D fabric material characterised with the aspects described herein and produced by a warp and/or weft knitting machines as is generally known in the art for example. In one illustrative embodiment, the at least one first layer 22 includes a network of fibres 40 acting as the spacer layer of the spacer fabric material formed from thermoplastic fibres or monofilaments interwoven or knitted with two fabric layers, as is generally known in the art.

The density and configuration of the network of fibres 40 within the third sub-layer 38 is selected to control the level of airflow 36 passing vertically through the first layer 22, as well as the support strength provided between the first sub-layer 30 and second sub-layer 32 when subjected to compressive loading. For example, a high density of fibres 40 may provide a more resilient support compared to a lower density of fibres 40. However, as the density of fibres 40 increases, the air flow 36 rate decreases between the first sub-layer 30 and the second sub-layer 32. A lower density of fibres 40 will provide reduced compressive resistance which may cause the first sub-layer 30 and the second sub-layer 32 to come together above a certain loading threshold, effectively collapsing the space 42 and blocking the air flow 36 between the first sub-layer 30 and a second sub-layer 32. The density of fibres 40 may be selected to provide an airflow 36 rate of 140 cubic centimeters per square centimeter of surface area of the first layer 22.

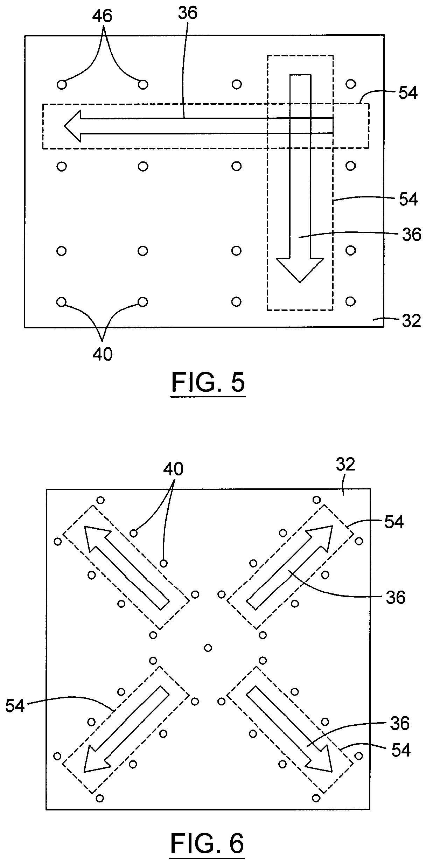

Now referring to FIG. 5 and FIG. 6, in addition to FIGS. 1 to 4, the network of fibres 40 may be aligned in horizontal rows along the width and/or length of the first layer 22 so as to form air flow channels 54 through which the air flow 36 may freely flow. Organizing and arranging the fibres 40 in such a way reduces resistance against which the air flow 36 may potentially encounter. While horizontal channels are illustrative, other channel shapes may also be formed by appropriately arranging the fibres 40. For example, the air flow channels 54 may be configured to radially extend from a center point of the multi-layered fabric 10 as shown in FIG. 6.

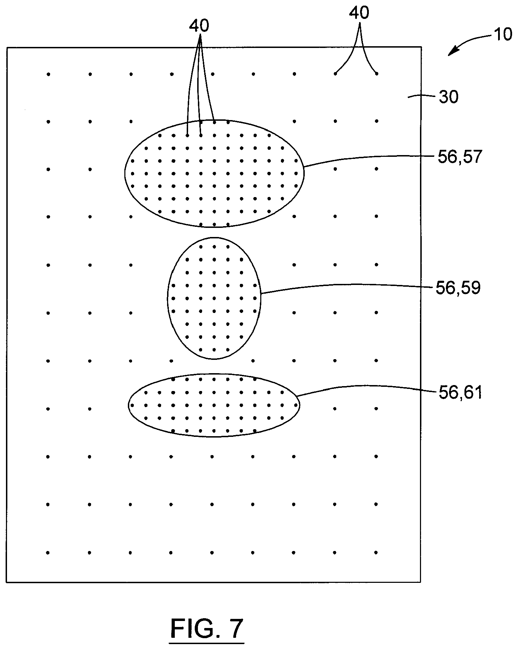

Now referring to FIG. 7, in addition to FIGS. 1 to 6, the network of fibres 40 may be dispersed between the first sub-layer 30 and the second sub-layer 32 in zones 56 of varying fibre 40 densities so as to provide varying degrees of load support to avoid the first sub-layer 30 and second sub-layer 32 from being compressed to the point where the first sub-layer 30 and second sub-layer 32 are brought together to pinch the air flow channels 54 closed, thereby removing any aeration effects of the multi-layered fabric 10. For example, when the multi-layered fabric 10 is constructed into a bed pad 16 as described in more detail hereinbelow to provide support to a bedridden patient, fibres 40 may be more densely concentrated about the center of the pad 16 so as to support the torso of the body 14 which tends to be heavier than a patient's extremities, such as their arms legs, and head. Optionally, a higher density of fibres 40 may be provided at a zone 57 of the pad 16 where the shoulders of a patient tend to rest. Optionally, a higher density of fibres 40 may be provided at a zone 59 that runs the length of the pad 16 to correspond with the lower back position of the body 14 when positioned on a bed 18. Optionally, a higher density of fibres 40 may be provided at a zone 61 that corresponds to the buttocks area of a patient.

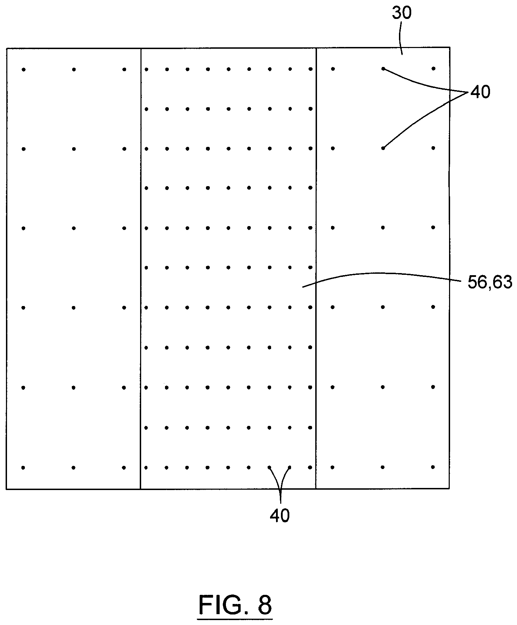

Now referring to FIG. 8, optionally, a higher density of fibres 40 may be provided at a zone 63 that runs the longitudinal length of the pad 16.

Fibre 40 densities between zones 57, 59, 61, 63 may vary and can change linearly or non-linearly depending on the desired support and aeration characteristics. The densities of the fibres 40 can be combined with the organization of air flow channels 54 to create the desired balance between support and aeration characteristics of the multi-layered fabric 10.

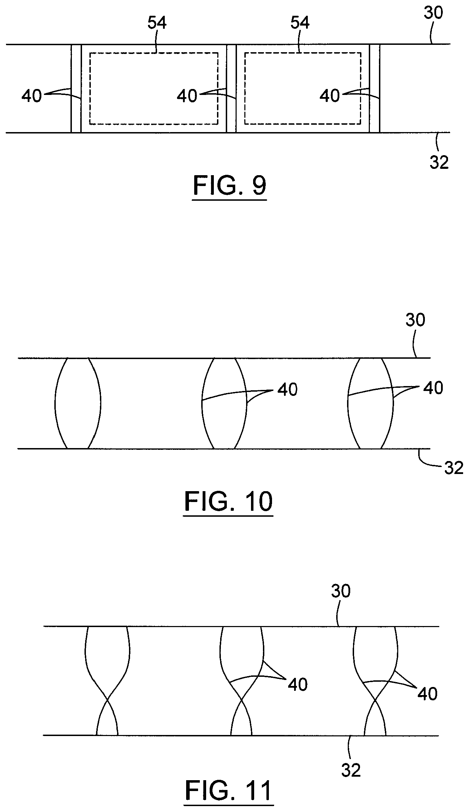

Now referring to FIGS. 9, 10 and 11, in addition to FIG. 3, the fibres 40 may be optionally disposed relative to the first sub-layer 30 and the second sub-layer 32 perpendicularly thereto, that is in a vertical direction. Optionally, the fibres 40 may be interconnected with the first sub-layer 30 and the second sub-layer 32 in a different configuration. For example, the fibres 40 may be connected to the first sub-layer 30 and the second sub-layer 32 in pairs as shown in FIG. 9 such that the air flow channels 54 are widened, with the pairs of fibres 40 still providing comparable support characteristics between the first sub-layer 30 and the second sub-layer 32 to that of a network of individual fibres 40 positioned evenly apart. Optionally, the pairs of fibres 40 may be biased to have a natural curvature which concaves away from each adjacent fibre 40 to form a spring action between the first sub-layer 30 and the second sub-layer 32 as illustrated in FIG. 10. Optionally, the pairs of fibres 40 may be twisted relative to one another to form a spring coil action between the first sub-layer 30 and the second sub-layer 32 as illustrated in FIG. 11.

The pairing of the fibres 40 to enhance the structural integrity of the first layer 22 can result in a multi-layered fabric 10 that comprises less fibres 40 but with an enhanced air flow 36 between the first sub-layer 30 and the second sub-layer 32 by reducing the number of fibres 40 the airflow 36 my encounter resistance against. Furthermore, the widening of the airflow channels 54 allows larger exudates to be trapped and allows for fluid 20 to pass with less resistance through the first layer 22. Of note, while pairs of fibres 40 are illustratively shown, other combinations such as triplets or quadruplets of fibres 40 may be provided.

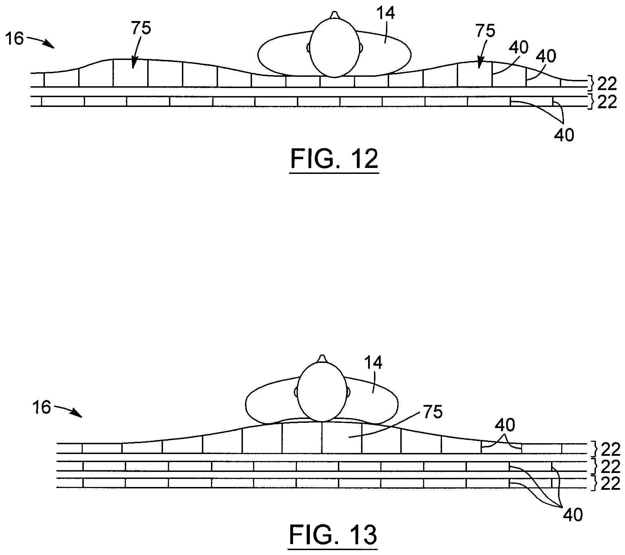

Now referring to FIG. 12 and FIG. 13, in addition to FIGS. 1, 2 and 3, optionally, the height of the third sub-layer 38 and fibre 40 distribution of each of the first sub-layer 22 may include different configurations. For example, the third sub-layer 38 height may be consistent or variable across the width and length of the first sub-layer 22. For example, the third sub-layer 38 height may be increased at areas of the multi-layered fabric 10 to naturally conform to the curvature surfaces of the body 14, such as the sides of the torso, to provide side support and increase comfort to the patient, as illustrated in FIG. 12. Alternatively, the third sub-layer 38 height may be increased in areas subject to higher compressive loading, for example along the center of a pad 16 to allow greater compression of the at least one first layer 22 while maintaining the spaces 42 which define the air flow channels 54. Protusions 75 may be formed by varying the height in such a manner, or by layering additional first layers 22 as will be described hereinbelow.

Still referring back to FIGS. 1 and 2, the second layer 24 is illustratively made of non-permeable material, so as to form a moisture and strikethrough barrier layer to capture or pool fluid 20 there above and thereupon and to prevent fluid 20 from seeping and entering into contact with a support, such as the bed 18, to soil it. Illustratively, the second layer 24 may be formed from a non-permeable material such as vinyl or nylon.

Still referring to FIGS. 1 and 2, there is illustratively provided an absorptive layer 58 provided in parallel and between the first layer 22 and second layer 24, and secured thereto to provide the multi-layered fabric 10 with fluid retention properties if desired depending on the type of wound to be managed. The absorptive layer 58 may be formed, for example, from a thin microfiber fabric that is fluid absorptive so as to assist with the wicking and absorption of fluid 20 away from at least one first layer 22. Depending on the medical application, the absorptive layer 58 can assist with maintaining a level of humidity below the skin 12 so as to promote healing as dependent on the medical treatment desired. As the absorptive layer 58 is illustratively provided away from the skin 12, such that the fluid 20 that is drawn away from the skin 12 can be collected and absorbed thereby.

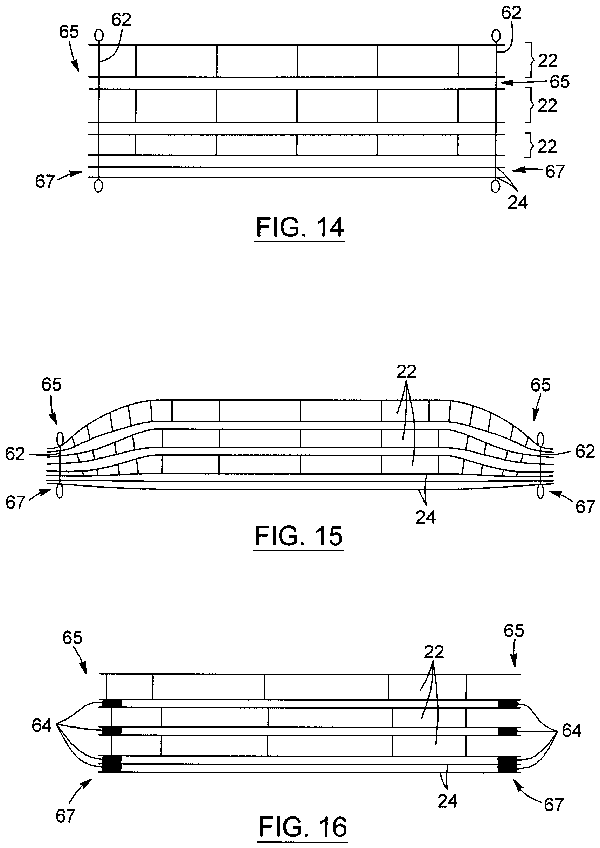

Now referring to FIG. 14, FIG. 15, and FIG. 16, in addition to FIG. 2, the at least one first layer 22 and at least one second layer 24 are attached together around their outer first layer periphery 65 and the second layer periphery 67. In accordance with an embodiment, the first layer 22 and at least one second layer 24 may be attached together with a stitching 62 stitched about the first layer periphery 65 and the second layer periphery 67. Illustratively the stitching 62 is a cotton or nylon thread, but other material types may be employed. Optionally, the outer first layer periphery 65 and the second layer periphery 67 may be compressively stitched together so as to bring the at least one first layer 22 and at least one second layer 24 together so as to compress and deform the at least one first layer 22 and at least one second layer 24 about the area of the outer first layer periphery 65 and the second layer periphery 67, as illustrated in FIG 15. Alternatively, the one first layer 22 and at least one second layer 24 are attached together around their outer first layer periphery 65 and the second layer periphery 67 using an adhesive 64, for example, or other form of bonding, as illustrated in FIG. 16.

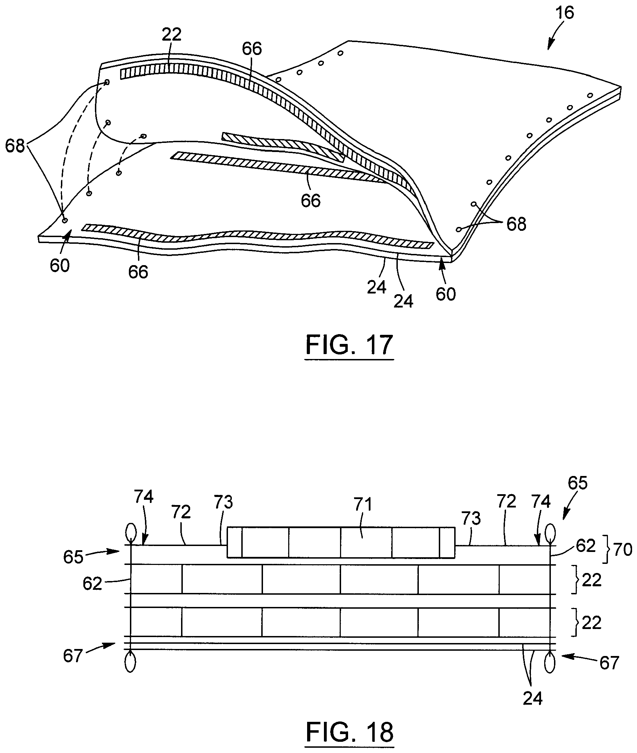

Now referring to FIG. 17, in addition to FIGS. 1 and 2, alternatively, the first layer 22 and at least one second layer 24 may be attached together around their outer first layer periphery 65 and the second layer periphery 67 using a removable fastener, such as VELCRO^{™} strips 66, or snaps 68, for example, which are provided about the outer first layer periphery 65 and the second layer periphery 67. Of note, the removable fastener, such as VELCRO^{™} strips 66, or snaps 68 may also be provided at other positions between the layers 22, 24, for example a VELCRO^{™} strip 66 may be provided down the middle of the multi-layered fabric 10. Removably securing the at least one first layer 22 and the at least one second layer 24 together may provide flexibility for adding or removing first layers 22 and at least one second layers 24 to the multi-layered fabric 10. For example, additional first layers 22 may be secured for creating a pad 16 for providing support to a patient that has a greater mass or for comfort reasons, or first layers 22 may be likewise removed for a patient that has a lessor mass or for applications which require a lessor number of first layers 22. Also a multi-layered fabric 10 may be disassembled such that the first layers 22 and at least one second layer 24 may be washed separately for more effective cleaning, or the layers 22, 24 may be individually replaced should a layer become worn. Also a multi-layered fabric 10 may be disassembled and reassembled with different first layers 10, for example for interchanging first layers 22 that comprise different thicknesses or different cross-sections depending on the wound management therapy contemplated.

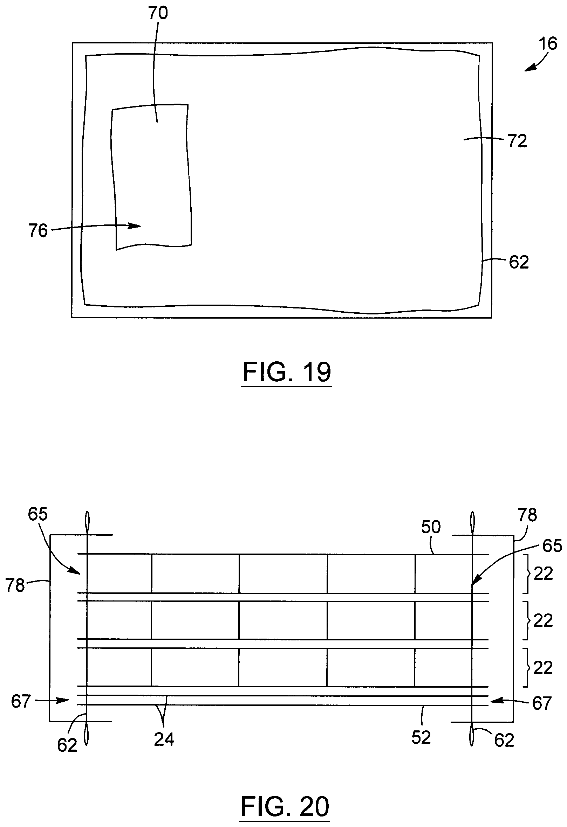

Now referring to FIG. 18 and FIG. 19, in addition to FIGS. 1 and 2, one or more fourth layers 70 including a sheet of material 72 connected to a smaller sized one first layers 22 forming a fifth layer 71 at a sheet inner periphery 73, may be provided. The sheet peripheral edge 74 may be connected to the outer peripheral edges 65 of an adjacent first layer 22 using stitching 62, VELCRO^{™} strips 66, or snaps 68 for example. With such a configuration portions of the multi-layered fabric 10 can be built up by layering smaller sized fifth layers 71 at different positions of the multi-layered fabric 10. For example, one or more smaller sized one first layers 70 can be stacked atop two first layers 22 as illustrated. The connection of the sheet peripheral edges 74 ensures that the fifth layer 71 remains properly positioned without adding height to the multi-layered fabric 10 at areas around its outer pad peripheries 60 for example. Variable heights of the multi-layered fabric 10 can thus be built up in this manner. For example, when the multi-layered fabric 10 is used to form a bed pad 18, one or more fourth layers 70 can be added in such a way so as to create an integrated pillow 76, or a pillow case.

Now referring to FIG. 20, in addition to FIGS. 2 and 17, the at least one first layer 22 and at least one second layer 24 are attached together around their first layer periphery 65 and the second layer periphery 67 with a breathable fabric peripheral cap 78 that overlaps an edge portion of the top layer 26 and the bottom layer 28 . The first layer 22 and the at least one second layer 24 may be attached together along with the breathable fabric peripheral cap 78 using stitching 62 stitched about the outer first layer periphery 65 and the second layer periphery 67 in a compressive or non-compressive fashion as described hereinabove manner. When stitched in a non-compressive manner, the at least one first layer 22 remains un-deformed and the air channels 54 remain opened at the first layer periphery 65 and the second layer periphery 67 thereof. Optionally, the breathable fabric peripheral cap 78 may include VELCRO^{™} strips 66, or snaps 68 provided on the top layer 26 and the bottom layer 28 to connect the breathable fabric peripheral cap 78 to the at least one first layer 22 and at least one second layer 24.

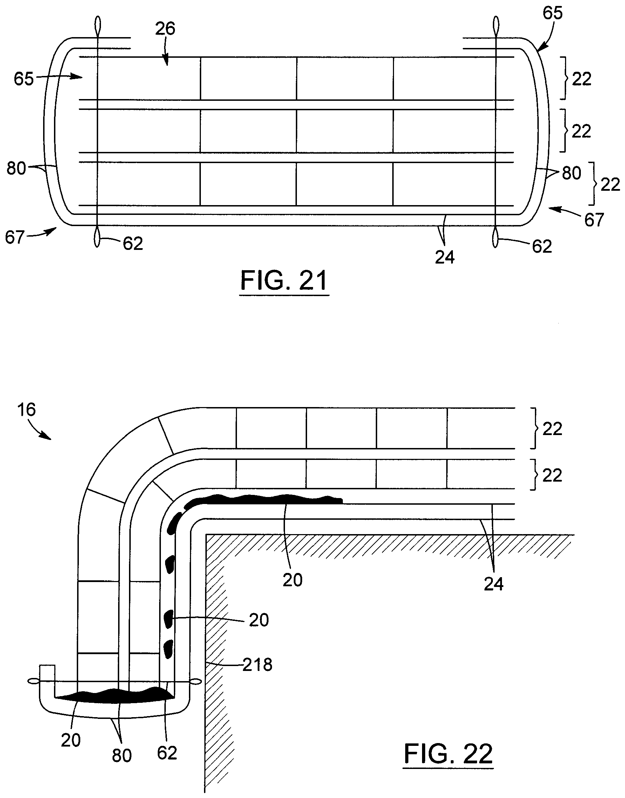

Now referring to FIG. 21, in addition to FIGS. 1 and 2, in accordance with an illustrative embodiment, the at least one first layer 22 and at least one second layer 24 are attached together around their outer peripheries 65, 67 with an overlapping portion 80 of the at least one second layer 24 that overlaps the top layer 26. With such a configuration, any fluid 20 which may be collected may remain contained within the multi-layered fabric 10 should the pad 16 be tilted, for example.

Now referring to FIG. 22 in addition to FIGS. 1, 2 and 21, in accordance with an illustrative embodiment, the first layer 22 and at least one second layer 24 are sized so as to overhang from the edge of the bed 18. In such an application, the overlapping portion 80 acts as a collector for any fluid 20 which may be shifted over the edge of the bed 18.

Still referring to FIGS. 1, 2, 3, and 4, the multi-layered fabric 10 can further be configured to provide certain moisture retention or moisture vapour transmission properties below the skin 12, which in some wound management treatments may be beneficial. For example, the number of first layers 22 may be selected to maintain fluid 20 at a distance from the skin 12 while the density of the network of fibres 40 maybe selected to control the evaporation rate, and the use of one or more layers of absorptive layers 50 can be used to manage ulcers with high discharge rate of exudates. Also, the number of air holes 34 can be selected to provide an airflow rate less than 140 cubic centimeters per square centimeter through the first layer 22.

The multi-layered fabric 10 can be washed to remove any exudates and fluid 20 that might have been collected or solidified within the spaces 42. Due to the organized and arranged network of fibres 40, water is able to easily penetrate the first layers 22 for cleaning of the spacers 42. Due to the hydrophobic structure of the multi-layered fabric 10, during washing water is prevented from being absorbed by the layers 22, 24 which avoids the multi-layered fabric 10 from becoming saturated and heavy. The organized and arranged network of fibres 40, as well as the permeability of the one first layer 22 also promotes airflow.

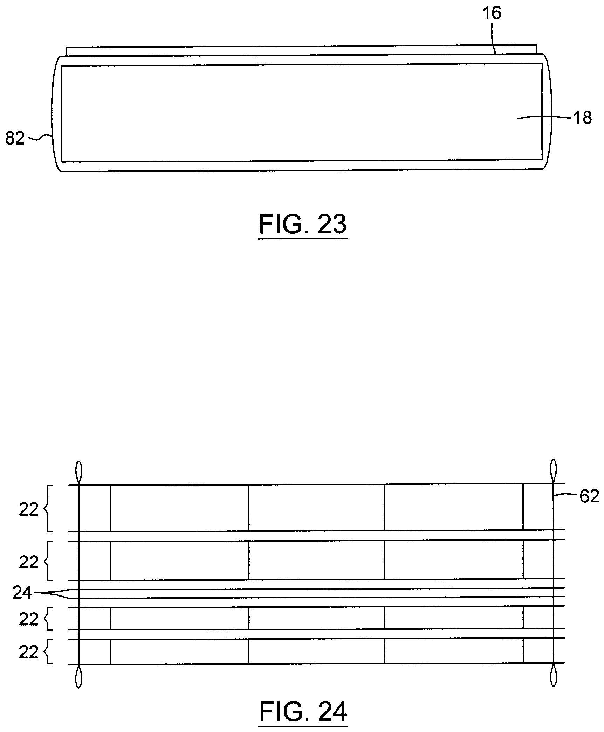

Now referring to FIG. 23, in addition to FIGS. 1 and 2, in accordance with an embodiment, the bed pad 18 further includes at least one elastic band 82 extending between two of the outer pad peripheries 60 and beneath the bed 16 so as to securely retain the bed pad 18 to the bed 16. In accordance with an embodiment, the bed pad 18 further comprises a sheet of material (not shown) secured to the outer pad periphery 60 of the bed pad 16 to engage a periphery of a bed 18, for example using an elastic edge as is known in the art for bed sheets.

Now referring to FIG. 24, in addition to FIGS. 1 and 2, in accordance with an embodiment of the present invention there is provided a multi-layered fabric 10 including additional at least one first layers 22 made of a permeable material disposed in parallel and secured under the at least one second layer 24. With such a configuration, additional support from the additional one first layers 22 may be employed without affecting the humidity levels beneath the skin 12.

While the present invention has been illustrated hereinabove with reference to a multi-layered fabric formed into a bed pad 16, other potential uses of the multi-layered fabric 10 may be possible, including wheelchair pads, pads for operating tables, pads for infant cribs, and other medical applications where absorption and cushioning properties are desired for the treatment of wounds. For example, the multi-layered fabric 10 may be formed into a pillow, a pillow case, or a liner for a casing such as a foot or arm support.

The multi-layered fabric 10 can thus be employed to treat exudate related conditions for preventative long term disabled care applications, and assist in the healing of existing bed ulcer conditions by enhancing airflow beneath a patient and by providing cushioned pressure relief support. The multi-layered fabric 10 can thus be employed to pre-emptively prevent exudate-related conditions. When used to treat exudate related conditions, the multi-layered fabric 10 can assist in containing leakage or exudate and prevent soiling of the support beneath the patient. The multi-layered fabric 10 can thus assist in relieving pain and discomfort to the patient suffering from decubitus ulcers and help reduce and/or control infection of a wound. The cleaning of the multi-layered fabric 10 is enhanced by providing a structure including a network of fibres 40 which allows water and airflow to easily penetrate to allow for a quick drying. Furthermore, the multilayered fabric 10 may be easily assembled and disassembled to replace, add or remove layers for adapting to specific wound management treatments.

Now referring back to FIG. 3, the plurality of fibers 40 are configured to form air channels 54 allowing air flow 36 to flow between the first sublayer 30 and the second sublayer 32.

Now referring back to FIGS. 2 and 4, the first sublayer 30 and the second sublayer 32 include holes 48 allowing air flow 36 and liquid 20 to flow there through.

Now referring back to FIG. 3, the first sublayer 30 and second sublayer 32 are made of a flexible material.

Now referring back to FIG. 2, he multi-layered fabric 10 further includes at least one third layer 27 of permeable material disposed in parallel and secured to the at least one first layer 22 opposite to the at least one second layer 24. Optionally, the third layer 27 is a polyester material. Optionally, the third layer 27 includes a patterned surface.

Still referring to FIG. 2, optionally the first sublayer 30 and second sublayer 32 are formed from a polyester material. Optionally, the first sublayer 30 and second sublayer 32 are formed from a microfiber fabric. Optionally, the first sublayer 30 and second sublayer 32 are formed from a microfilament fabric. Optionally, the first sublayer 30 and second sublayer 32 are hydrophobic.

Still referring to FIG. 2, the at least one second layer 24 includes two second layers 24. Optionally, the at least one second layer 24 is formed from nylon. Optionally the at least one first layer 22 is a three dimensional spacer fabric. Optionally, the plurality of fibers 40 are disposed perpendicularly to first sublayer 30 and the second sublayer 32. Optionally the plurality of fibers 40 each comprise a bend. Optionally, the plurality of fibers 40 are aligned such that the fibers 40 bend in a common direction.

Now referring to FIGS. 9, 10, 11, optionally, the plurality of fibers 40 are disposed in pairs of fibers 40. Optionally, the pairs of fibers 40 are twisted.

Referring back to FIG. 2, optionally the plurality of fibers 40 are made of nylon. Optionally, the plurality of fibers 40 are made of polyester. Optionally, the plurality of fibers 40 are a monofilament fiber. The density of the plurality of fibres 40 allows a vertical airflow 36 of 140 cubic centimeters per square centimeter of surface area. Optionally, the first sublayer 30 and the second sublayer 32 are spaced apart by a distance of between three to twelve millimeters. Optionally, the first sublayer 30 and the second sublayer 32 are spaced apart by a distance that varies over the area of the at least one first layer 22. Optionally, the plurality fibers 40 are hydrophobic. Optionally, the fibers 40 are chemically treated to render the plurality of fibres 40 hydrophobic. Optionally, the plurality of fibers 40 are knitted to the first sublayer 30 and the second sublayer 32.

Now referring to FIG. 3, the first sublayer 30 and second sublayer 32 include bristles 48 protruding from a first sublayer surface 55 and a second sublayer surface 51 for providing frictional engagement with the bristles 48 of an adjacent sublayer 30,32. Optionally, the bristles 48 are formed in part by a portion of the fiber 40.

Now referring to FIG. 3, optionally, the multi-layered fabric 10 includes three first layers 22.

Now referring to FIG. 12 and FIG. 13, optionally, each first layer 22 has a different thickness than an adjacent first layer 22. Optionally, each first layer 22 has an increasing thickness to an adjacent first layer 22 wherein the first layer 22 adjacent the second layer 24 has a lessor thickness than a first layer 22 opposite the second layer 24.

Now referring to FIG. 2, the multi-layered fabric 10 is machine washable.

Now referring to FIG. 21, optionally the at least one second layer 24 extends to overlap with the at least one first layer 22.

Now referring to FIGS. 14, and 15, optionally, the at least one first layer 22 is secured to the at least one second layer 24 by stitching. Optionally, the at least one first layer 22 comprises a first layer periphery 65 and the at least one second layer 24 comprises a second layer periphery 67, wherein the first layer periphery 65 and the second layer periphery 67 are secured together by stitching. Optionally, the first layer periphery 65 and the second layer periphery 67 are compressively stitched together.

Now referring to FIG. 20, the multi-layered fabric 10 further includes a breathable fabric peripheral cap 78 secured to the first layer periphery 65 and the second layer periphery 67.

Now referring back to FIG. 1, the bed pad 16 includes the multi-layered fabric 10, wherein the bed pad 16 may include one or more outer pad peripheries 60. Optionally, the bed pad 16 includes at least one elastic band 82 extending between the one or more outer pad peripheries 60. Optionally, the bed pad 16 may include a sheet of material secured to the one or more outer pad peripheries 60 to engage a periphery of a bed 18. Optionally, two or more bed pads 16 may be provided with each including the multi-layered fabric 10 wherein each of the two or more bed pads 16 are secured together to create a foldable pad.

Now referring to FIGS. 12, 13, and 18, optionally, the bed pad 16, includes one or more fourth layers 70, each fourth layer 70 includes a fifth layer 71 including the first layer 22, and a sheet of material 72 including a sheet outer periphery 74 and a sheet inner periphery 73, wherein the sheet outer periphery 74 is connected to the at least one first layer 22 and the sheet inner periphery 73 is connected to the fifth layer 71, wherein the one or more fourth layers 70 is disposed in parallel to the at least one first layer 22 to form protrusions 75 matching the curves of a human body 14. Optionally, the bed pad 10 includes one or more fourth layers 70 to form protrusions 75 running longitudinally the length of the bed pad 16.

Now referring to FIG. 22, optionally, the bed pad 16 is sized to be larger than the surface of a hospital bed 18 such that a portion of the bed pad 16 overhangs the bed 18.

Although optional embodiments of the invention have been described in detail herein and illustrated in the accompanying drawings, it is to be understood that the invention is not limited to those precise embodiments and that various changes and modifications may be effected therein without departing from the scope of the claims.

## Claims

1. A multi-layered fabric (10) for use in padding for treating ulcers, said fabric comprising:
at least one first layer (22) comprising:
a first sublayer (30) made of permeable material;
a second sublayer (32) made of permeable material; and
a third sublayer (38) located between the first and the second sublayers (32), the third sublayer (38) comprising a plurality of fibers (40) interconnecting the first sublayer (30) with the second sublayer (32) for allowing an airflow (36) between the first and second sublayers (32); and
at least one second layer (24) made of impermeable material disposed in parallel and secured to the at least one first layer (22);
wherein the density of the plurality of fibers (40) allows a vertical airflow (36) of 140 cubic centimeters per square centimeter of surface area, the airflow (36) being a vertical airflow (36) through the first sublayer (30) and the second sublayer (32);and
wherein the first and second sublayers (32) comprise bristles (48) protruding from a first sublayer surface and a second sublayer surface for providing frictional engagement with the bristles (48) of an adjacent sublayer.

2. The multi-layered fabric (10) of claim 1, wherein the plurality of fibers (40) are configured to form air channels allowing air flow to flow between the first sublayer (30) and the second sublayer (32).

3. The multi-layered fabric (10) of claim 1 or 2, wherein the first sublayer (30) and the second sublayer (32) comprise holes (34) allowing air flow and liquid to flow therethrough; and wherein the first sublayer (30) and second sublayer (32) are made of a flexible material.

4. The multi-layered fabric (10) of any one of claims 1 to 3, further comprising at least one third layer (27) of permeable material disposed in parallel and secured to the at least one first layer (22) opposite to the at least one second layer (24).

5. The multi-layered fabric (10) of claim 4, wherein the third layer (27) is a polyester material; and wherein the third layer (27) comprises a patterned surface.

6. The multi-layered fabric (10) of any one of claims 1 to 5, wherein the first and second sublayers (32) are formed from either a polyester material, a microfiber fabric or a microfilament fabric.

7. The multi-layered fabric (10) of any one of claims 1 to 6, wherein the first and second sublayers (32) are hydrophobic.

8. The multi-layered fabric (10) of any one of claims 1 to 7, wherein the at least one second layer (24) comprises two second layers (24); and is formed from nylon.

9. The multi-layered fabric (10) of any one of claims 1 to 8, wherein the at least one first layer (22) is a three dimensional spacer fabric.

10. The multi-layered fabric (10) of any one of claims 1 to 9, wherein the plurality of fibers (40) are disposed perpendicularly to the first sublayer (30) and the second sublayer (32); wherein the plurality of fibers (40) each comprise a bend; wherein the plurality of fibers (40) are aligned such that the fibers (40) bend in a common direction; wherein the plurality of fibers (40) are disposed in pairs of fibers (40); and wherein the pairs of fibers (40) are twisted.

11. The multi-layered fabric (10) of any one of claims 1 to 10, wherein the plurality of fibers (40) are made either of nylon or polyester or wherein the fibers (40) are a monofilament fiber.

12. The multi-layered fabric (10) of any one of claims 1 to 11, wherein the first sublayer (30) and the second sublayer (32) are spaced apart by a distance of between three to twelve millimeters; and wherein the first sublayer (30) and the second sublayer (32) are spaced apart by a distance that varies over the area of the at least one first layer (22).

13. The multi-layered fabric (10) of any one of claim 1 to 12, wherein the plurality of fibers (40) are hydrophobic; wherein the plurality of fibers (40) are chemically treated to render the plurality of fibres (40) hydrophobic; wherein the plurality of fibers (40) are knitted to the first sublayer (30) and the second sublayer (32); wherein the bristles (48) are formed in part by a portion of the fibers (40); wherein the at least one first layer (22) comprises three first layers (22); wherein each first layer (22) has a different thickness than an adjacent first layer (22); and wherein each first layer (22) has an increasing thickness to an adjacent first layer (22) wherein the first layer (22) adjacent the second layer (24) has a lessor thickness than a first layer (22) opposite the second layer (24).

14. The multi-layered fabric (10) of any one of claims 1 to 13, wherein the fabric is machine washable; wherein the at least one second layer (24) extends to overlap with the at least one first layer (22); wherein the at least one first layer (22) is secured to the at least one second layer (24) by stitching; wherein the at least one first layer (22) comprises a first layer (22) periphery and the at least one second layer (24) comprises a second layer (24) periphery, wherein the first layer (22) periphery and the second layer (24) periphery are secured together by stitching; wherein the first layer (22) periphery and the second layer (24) periphery are compressively stitched together; and wherein the multi-layered fabric (10) further comprises a breathable fabric peripheral cap secured to the first layer (22) periphery and the second layer (24) periphery.

15. A bed pad (16) comprising the multi-layered fabric (10) of any one of claims 1 to 14, wherein the bed pad (16) comprises one or more outer pad peripheries (60); and/or wherein the bed pad (16) further comprises at least one elastic band (82) extending between the one or more outer pad peripheries (60); and/or wherein the bed pad (16) further comprises a sheet of material secured to the one or more outer pad peripheries (60) to engage a periphery of a bed (18); and/or wherein the bed pad (16) further comprises one or more fourth layers (70), each fourth layer (70) comprising: a fifth layer (71) comprising the first layer (22); and a sheet of material (72) comprising a sheet outer periphery (74) and a sheet inner periphery (73), wherein the sheet outer periphery (74) is connected to the at least one first layer (22) and the sheet inner periphery (73) is connected to the fifth layer (71); wherein the one or more fourth layers (70) is disposed in parallel to the at least one first layer (22) to form protrusions (75) matching the curves of a human body, preferably wherein the one or more fourth layers (70) are provided to form protrusions (75) running longitudinally the length of the bed pad (16); and/or wherein the bed pad (16) is sized to be larger than the surface of a hospital bed (18) such that a portion of the bed pad (16) overhangs the bed (18).

## Patentansprüche

1. Mehrschichtiger Stoff (10) zur Verwendung als Polsterung zum Behandeln von Geschwüren, wobei der besagte Stoff Folgendes umfasst:
mindestens eine erste Schicht (22), die Folgendes umfasst:
eine erste Teilschicht (30) aus durchlässigem Material;
eine zweite Teilschicht (32) aus durchlässigem Material; und
eine dritte Teilschicht (38), die sich zwischen der ersten und der zweiten Teilschicht (32) befindet, wobei die dritte Teilschicht (38) eine Vielzahl von Fasern (40) umfasst, die die erste Teilschicht (30) mit der zweiten Teilschicht (32) verbinden, um einen Luftstrom (36) zwischen der ersten und der zweiten Teilschicht (32) zu ermöglichen; und
mindestens eine zweite Schicht (24) aus undurchlässigem Material, die parallel angeordnet ist und an der mindestens einen ersten Schicht (22) befestigt ist;
wobei die Dichte der Vielzahl von Fasern (40) einen senkrechten Luftstrom (36) von 140 Kubikzentimetern pro Quadratzentimeter Oberfläche ermöglicht, wobei der Luftstrom (36) ein senkrechter Luftstrom (36) durch die erste Teilschicht (30) und die zweite Teilschicht (32) ist; und
wobei die erste und die zweite Teilschicht (32) Borsten (48) umfassen, die von einer ersten Teilschichtoberfläche und einer zweiten Teilschichtoberfläche vorstehen, um einen Reibungseingriff mit den Borsten (48) einer benachbarten Teilschicht bereitzustellen.

2. Mehrschichtiger Stoff (10) nach Anspruch 1, wobei die Vielzahl von Fasern (40) konfiguriert sind, um Luftkanäle zu bilden, die einem Luftstrom ermöglichen, zwischen der ersten Teilschicht (30) und der zweiten Teilschicht (32) zu strömen.

3. Mehrschichtiger Stoff (10) nach Anspruch 1 oder 2, wobei die erste Teilschicht (30) und die zweite Teilschicht (32) Löcher (34) umfassen, die einem Luftstrom und einer Flüssigkeit ermöglichen, dadurch zu strömen; und wobei die erste Teilschicht (30) und die zweite Teilschicht (32) aus einem flexiblen Material sind.

4. Mehrschichtiger Stoff (10) nach einem der Ansprüche 1 bis 3, der ferner mindestens eine dritte Schicht (27) aus durchlässigem Material umfasst, die parallel angeordnet ist und an der mindestens einen ersten Schicht (22) gegenüber der mindestens einen zweiten Schicht (24) befestigt ist.

5. Mehrschichtiger Stoff (10) nach Anspruch 4, wobei die dritte Schicht (27) ein Polyestermaterial ist; und wobei die dritte Schicht (27) eine gemusterte Oberfläche umfasst.

6. Mehrschichtiger Stoff (10) nach einem der Ansprüche 1 bis 5, wobei die erste und die zweite Teilschicht (32) entweder aus einem Polyestermaterial, einem Mikrofaserstoff oder einem Mikrofilamentstoff gebildet sind.

7. Mehrschichtiger Stoff (10) nach einem der Ansprüche 1 bis 6, wobei die erste und die zweite Teilschicht (32) hydrophob sind.

8. Mehrschichtiger Stoff (10) nach einem der Ansprüche 1 bis 7, wobei die mindestens eine zweite Schicht (24) zwei zweite Schichten (24) umfasst; und aus Nylon gebildet ist.

9. Mehrschichtiger Stoff (10) nach einem der Ansprüche 1 bis 8, wobei die mindestens eine erste Schicht (22) ein dreidimensionaler Abstandsstoff ist.

10. Mehrschichtiger Stoff (10) nach einem der Ansprüche 1 bis 9, wobei die Vielzahl von Fasern (40) senkrecht zu der ersten Teilschicht (30) und der zweiten Teilschicht (32) angeordnet sind; wobei die Vielzahl von Fasern (40) jeweils eine Biegung umfassen; wobei die Vielzahl von Fasern (40) so ausgerichtet sind, dass sich die Fasern (40) in eine gemeinsame Richtung biegen; wobei die Vielzahl von Fasern (40) in Faserpaaren (40) angeordnet sind; und wobei die Faserpaare (40) verdreht sind.

11. Mehrschichtiger Stoff (10) nach einem der Ansprüche 1 bis 10, wobei die Vielzahl von Fasern (40) entweder aus Nylon oder Polyester sind oder wobei die Fasern (40) eine Monofilamentfaser sind.

12. Mehrschichtiger Stoff (10) nach einem der Ansprüche 1 bis 11, wobei die erste Teilschicht (30) und die zweite Teilschicht (32) durch einen Abstand von zwischen drei bis zwölf Millimetern beabstandet sind; und wobei die erste Teilschicht (30) und die zweite Teilschicht (32) durch einen Abstand beabstandet sind, der über den Bereich der mindestens einen ersten Schicht (22) variiert.

13. Mehrschichtiger Stoff (10) nach einem der Ansprüche 1 bis 12, wobei die Vielzahl von Fasern (40) hydrophob sind; wobei die Vielzahl von Fasern (40) chemisch behandelt sind, um die Vielzahl von Fasern (40) hydrophob zu machen; wobei die Vielzahl von Fasern (40) mit der ersten Teilschicht (30) und der zweiten Teilschicht (32) verstrickt sind; wobei die Borsten (48) teilweise durch einen Teil der Fasern (40) gebildet sind; wobei die mindestens eine erste Schicht (22) drei erste Schichten (22) umfasst; wobei jede erste Schicht (22) eine andere Dicke als eine benachbarte erste Schicht (22) hat; und wobei jede erste Schicht (22) eine zunehmende Dicke zu einer benachbarten ersten Schicht (22) hat, wobei die erste Schicht (22), die benachbart zu der zweiten Schicht (24) ist, eine geringere Dicke als eine erste Schicht (22) gegenüber der zweiten Schicht (24) hat.

14. Mehrschichtiger Stoff (10) nach einem der Ansprüche 1 bis 13, wobei der Stoff maschinenwaschbar ist; wobei sich die mindestens eine zweite Schicht (24) erstreckt, um mit der mindestens einen ersten Schicht (22) zu überlappen, wobei die mindestens eine erste Schicht (22) durch Nähen an der mindestens einen zweiten Schicht (24) befestigt ist; wobei die mindestens eine erste Schicht (22) einen Umfang der ersten Schicht (22) umfasst und die mindestens eine zweite Schicht (24) einen Umfang der zweiten Schicht (24) umfasst, wobei der Umfang der ersten Schicht (22) und der Umfang der zweiten Schicht (24) durch Nähen aneinander befestigt sind; wobei der Umfang der ersten Schicht (22) und der Umfang der zweiten Schicht (24) unter Druck miteinander vernäht sind; und wobei der mehrschichtige Stoff (10) ferner eine Randkappe aus atmungsaktivem Stoff umfasst, die an dem Umfang der ersten Schicht (22) und dem Umfang der zweiten Schicht (24) befestigt ist.

15. Bettpolster (16), das den mehrschichtigen Stoff (10) nach einem der Ansprüche 1 bis 14 umfasst, wobei das Bettpolster (16) einen oder mehrere äußere Polsterumfänge (60) umfasst; und/oder wobei das Bettpolster (16) ferner mindestens ein elastisches Band (82) umfasst, das sich zwischen dem einen oder den mehreren äußeren Polsterumfängen (60) erstreckt; und/oder wobei das Bettpolster (16) ferner eine Materialbahn umfasst, die an dem einen oder den mehreren äußeren Polsterumfängen (60) befestigt ist, um mit einem Umfang eines Bettes (18) in Eingriff zu kommen; und/oder wobei das Bettpolster (16) ferner eine oder mehrere vierte Schichten (70) umfasst, wobei jede vierte Schicht (70) Folgendes umfasst: eine fünfte Schicht (71), die die erste Schicht (22) umfasst; und eine Materialbahn (72), die einen Bahnaußenumfang (74) und einen Bahninnenumfang (73) umfasst, wobei der Bahnaußenumfang (74) mit der mindestens einen ersten Schicht (22) verbunden ist und der Bahninnenumfang (73) mit der fünften Schicht (71) verbunden ist; wobei die eine oder die mehreren vierten Schichten (70) parallel zu der mindestens einen ersten Schicht (22) angeordnet sind, um Vorsprünge (75) zu bilden, die mit den Kurven eines menschlichen Körpers übereinstimmen, wobei vorzugsweise die eine oder die mehreren vierten Schichten (70) vorgesehen sind, um Vorsprünge (75) zu bilden, die in Längsrichtung über die Länge des Bettpolsters (16) verlaufen; und/oder wobei das Bettpolster (16) bemessen ist, um größer als die Oberfläche eines Krankenhausbettes (18) zu sein, so dass ein Teil des Bettpolsters (16) über das Bett (18) hinausragt.

## Revendications

1. Tissu multicouche (10) pour l'utilisation dans un rembourrage pour traiter les ulcères, ledit tissu comprenant :
au moins une première couche (22) comprenant :
une première sous-couche (30) en matériau perméable ;
une deuxième sous-couche (32) en matériau perméable ; et
une troisième sous-couche (38) située entre la première et la deuxième sous-couches (32), la troisième sous-couche (38) comprenant une pluralité de fibres (40) interconnectant la première sous-couche (30) avec la deuxième sous-couche (32) pour autoriser un flux d'air (36) entre la première et deuxième sous-couches (32) ; et
au moins une deuxième couche (24) en matériau imperméable disposée parallèlement et sécurisée à au moins une première couche (22) ;
où la densité de la pluralité de fibres (40) permet un flux d'air vertical (36) de 140 centimètres cubiques par centimètre carré de surface, le flux d'air (36) étant un flux d'air vertical (36) à travers la première sous-couche (30) et la deuxième sous-couche (32) ; et
où les première et deuxièmes sous-couches (32) comprennent des poils (48) saillants d'un première surface de sous-couche et une deuxième surface de sous-couche pour fournir un engagement par friction avec les poils (48) d'une sous-couche adjacente.

2. Tissu multicouche (10) selon la revendication 1, où la pluralité de fibres (40) sont configurées pour former des canaux d'air permettant au flux d'air de circuler entre la première sous-couche (30) et la deuxième sous-couche (32).

3. Tissu multicouche (10) selon la revendication 1 ou 2, où la première sous-couche (30) et la deuxième sous-couche (32) comprennent des trous (34) permettant au flux d'air et au liquide de circuler à travers ; et où la première sous-couche (30) et la deuxième sous-couche (32) sont faites d'un matériau flexible.

4. Tissu multicouche (10) selon l'une quelconque des revendications 1 à 3, comprenant en outre au moins une troisième couche (27) d'un matériau perméable disposée parallèlement et sécurisée à au moins une première couche (22) opposée à au moins une deuxième couche (24).

5. Tissu multicouche (10) selon la revendication 4, où la troisième couche (27) est un matériau polyester ; et où la troisième couche (27) comprend une surface à motifs.

6. Tissu multicouche (10) selon l'une quelconque des revendications 1 à 5, où la première et la deuxième sous-couches (32) sont formées soit d'un matériau polyester, soit d'un tissu microfibre ou soit d'un tissu de microfilaments.

7. Tissu multicouche (10) selon l'une quelconque des revendications 1 à 6, où la première et la deuxième sous-couches (32) sont hydrophobes.

8. Tissu multicouche (10) selon l'une quelconque des revendications 1 à 7, où l'au moins une deuxième couche (24) comprend deux deuxièmes couches (24) ; et est formée de nylon.

9. Tissu multicouche (10) selon l'une quelconque des revendications 1 à 8, où l'au moins une première couche (22) est un tissu d'espacement tridimensionnel.

10. Tissu multicouche (10) selon l'une quelconque des revendications 1 à 9, où la pluralité de fibres (40) sont disposées perpendiculairement à la première sous-couche (30) et à la deuxième sous-couche (32) ; où la pluralité de fibres (40) chacune comprend un pliage ; où la pluralité de fibres (40) sont alignées de sorte que les fibres (40) se plient dans une direction commune ; où la pluralité de fibres (40) sont disposées par paires de fibres (40) ; et où les paires de fibres (40) sont torsadées.

11. Tissu multicouche (10) selon l'une quelconque des revendications 1 à 10, où la pluralité de fibres (40) sont faites soit de nylon soit de polyester ou où les fibres (40) sont une fibre monofilament.

12. Tissu multicouche (10) selon l'une quelconque des revendications 1 à 11, où la première sous-couche (30) et la deuxième sous-couche (32) sont espacées par une distance entre trois et douze millimètres ; et où la première sous-couche (30) et la deuxième sous-couche (32) sont espacées par une distance qui varie en fonction de la surface d'au moins une première couche (22).

13. Tissu multicouche (10) selon l'une quelconque des revendication 1 à 12, où la pluralité de fibres (40) sont hydrophobes ; où la pluralité de fibres (40) sont traitées chimiquement pour rendre la pluralité de fibres (40) hydrophobes ; où la pluralité de fibres (40) sont tricotées à la première sous-couche (30) et à la deuxième sous-couche (32) ; où les poils (48) sont formés en partie par une portion de fibres (40) ; où au moins une première couche (22) comprend trois premières couches (22) ; où chaque première couche (22) a une épaisseur différente à celle d'une première couche adjacente (22) ; et où chaque première couche (22) a une épaisseur croissante par rapport à une première couche adjacente (22) où la première couche (22) adjacente à la deuxième couche (24) a une épaisseur inférieure à une première couche (22) opposée à la deuxième couche (24).

14. Tissu multicouche (10) selon l'une quelconque des revendications 1 à 13, où le tissu est lavable en machine ; où au moins une deuxième couche (24) s'étend pour chevaucher au moins une première couche (22) ; où au moins une première couche (22) est fixée à au moins une deuxième couche (24) par couture ; où au moins une première couche (22) comprend une périphérie de la première couche (22) et au moins une deuxième couche (24) comprend une périphérie de la deuxième couche (24), où la périphérie de la première couche (22) et la périphérie de la deuxième couche (24) sont fixées ensemble par couture ; où la périphérie de la première couche (22) et la périphérie de la deuxième couche (24) sont cousues ensemble de manière compressive ; et où le tissu multicouche (10) comprend en outre une couverture périphérique de tissu respirant fixée à la périphérie de première couche (22) et à la périphérie de la deuxième couche (24).

15. Alaise de lit (16) comprenant le tissu multicouche (10) selon l'une quelconque des revendications 1 à 14, où l'alaise de lit (16) comprend un ou plusieurs périphéries extérieures d'alaise (60) ; et/ou où l'alaise de lit (16) comprend en outre au moins une bande élastique (82) s'étendant entre l'une ou plusieurs périphéries extérieures d'alaise (60) ; et/ou où l'alaise de lit (16) comprend en outre une feuille de matériau fixée à l'une ou plusieurs périphéries extérieures d'alaise (60) pour engager une périphérie d'un lit (18) ; et/ou où l'alaise de lit (16) comprend en outre une ou plusieurs quatrièmes couches (70), chaque quatrième couche (70) comprenant : une cinquième couche (71) comprenant la première couche (22) ; et une feuille de matériau (72) comprenant une périphérie extérieure de la feuille (74) et une périphérie intérieure de la feuille (73), où la périphérie extérieure de la feuille (74) est connectée à au moins une première couche (22) et la périphérie intérieure de la feuille (73) est connectée à la cinquième couche (71) ; où l'une ou plusieurs quatrièmes couches (70) sont disposées parallèlement à au moins une première couche (22) pour former des protubérances (75) correspondant aux courbes d'un corps humain, préférablement où l'une ou plusieurs quatrièmes couches (70) sont prévues pour former des protubérances (75) s'étendant longitudinalement sur la longueur de l'alaise de lit (16) ; et/ou où l'alaise de lit (16) est dimensionné pour être plus grand que la surface d'un lit d'hôpital (18) de telle sorte qu'une partie de l'alaise de lit (16) surplombe le lit (18).
